# EUROPEAN PATENT APPLICATION

(11) **EP 1 916 017 A1**
(43) Date of publication of application: **30.04.2008**
(21) Application number: 07119147.2
(22) Date of filing: 24.10.2007
(51) Int. Cl.: A61N 5/06, F21V 17/10

(54) **Device for supporting filters for sunlamps**

(30) Priority: 27.10.2006 IT GE20060020 U
(71) Applicant: Tecnova di Ciccarello Spitaleri Angelo, 16167 Genova (IT)
(72) Inventor: Ciccarello Spitaleri, Angelo, 16167, Genova (IT)
(74) Representative: Porsia, Attilio

(57) **Abstract**

Support device for sun lamp filters used in tanning apparatus, comprising, for each lamp group (2), frame means (103, 403; 603) for supporting at least one filter (203, 303), said supporting frame means (103, 403; 603) being hinged along one side with the structural frame (201) of said apparatus, and said frame (103, 403; 603) for supporting the said filters being movable with respect to said structural frame of said apparatus through a given angle.

## Description

The present invention relates to tanning sun lamps and in particular relates to a device for supporting the filters used in said lamps.

In tanning apparatus, both for home use and for professional use, each of the lamps present must be equipped with one or more filters able to reduce the frequency of the UV rays emitted by the lamp and potentially dangerous for the user. This filter normally consists of a panel made of glass or similar suitably coloured material; typically, cobalt glass filters with a characteristic blue-violet colour are widely used. The filters are normally mounted on support frames which are in turn fixed by means of screws or the like to the structure of the apparatus. The deposition of dust on the filters reduces considerably the functional efficiency of the lamps and therefore the filters themselves must be periodically removed in order to carry out maintenance thereof, which therefore requires somewhat complex disassembly operations. Moreover, only with removal of the filter is it possible to access the lamp and the parabolic reflector.

In some cases, as for example in the case of so-called sun beds, it must also be considered that the lamps are oriented perpendicularly with respect to the horizontal plane and therefore the operation of disassembly and removal of the filters is even more complex. Moreover, if it is considered that generally this type of apparatus comprises at least one column with three to six lamps arranged above each other, the maintenance operations become really complex and require a very long time.

The object of the present invention is to provide a support device for sun lamp filters used in tanning apparatus, which allows simple and immediate access both to the filters and to the lamp and which at the same time ensures a stable and orderly arrangement of the filters. The abovementioned tanning apparatus comprise beds/showers for integral tanning, and three-sided, four-sided and six-sided lamps for tanning the face and the neck and shoulders. The lamps of the beds and showers are mounted on various columns in a number varying from 5 to 7 and are aligned in the longitudinal direction; in other apparatus, the lamps are aligned horizontally or arranged on top of each other. The object of the present invention, therefore, is to provide a solution which is also applicable to single lamps.

The present invention therefore relates to a support device for sun lamp filters used in tanning apparatus, comprising, for each lamp group, frame means for supporting at least one filter, said support means being hinged along one side with the structural frame of said apparatus, and said support frame for said filters being movable with respect to said structural frame of said apparatus through a given angle.

In a preferred embodiment, all the filter support frames of said lamps are connected together by means of a rigid connection means so that the displacement of first support frame causes the displacement of all the support frames. Advantageously, means are envisaged for locking at least one of the support frames so as to prevent accidental opening.

In particular, the support frame may consist of two sections, for example in the form of a double C, inside which two opposite sides of the filters are inserted. A bracket is fixed to one of the ends of each of the said sections and has an end flange perpendicular to the plane of the filters, while the bracket fixing mean acts as a pivot for hinging the support frame thus designed on the structural frame of the apparatus.

Further advantages and characteristic features will become clear from the following description of a few embodiments of the device according to the present invention, in which:
Figure 1 is a partially longitudinally sectioned view of a tanning apparatus provided with the device according to the present invention, in the closed configuration;
Figure 2 is a view similar to that of Figure 1, with the device in the open configuration;
Figure 3 is a perspective view of a detail according to Figure 2;
Figure 4 is a cross-sectional view of a constructional variant of the present invention; and
Figure 5 is a perspective view, with parts cross-sectioned, of a detail of Figure 4.

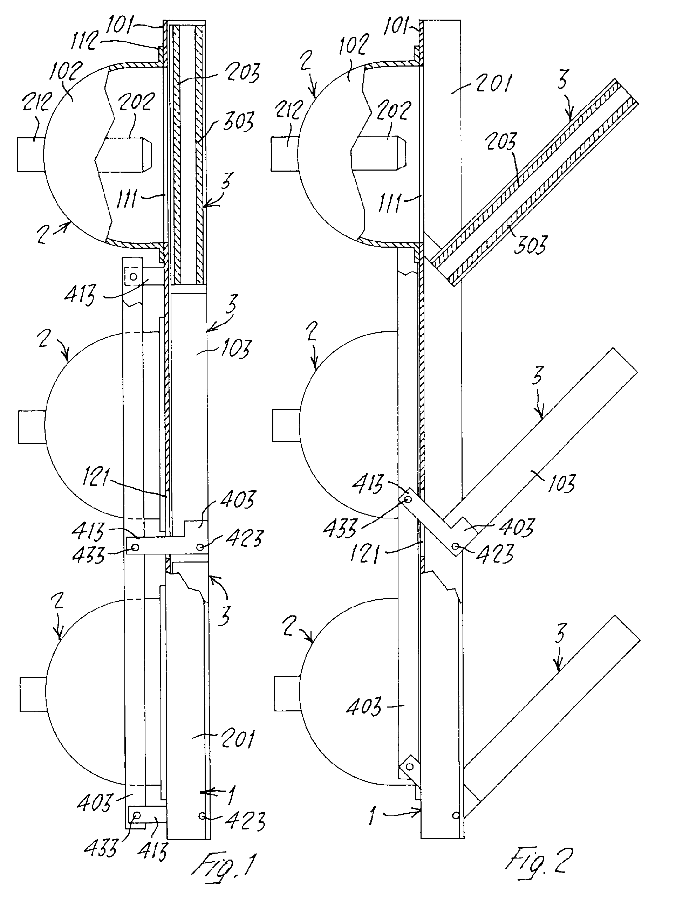

Figure 1 shows a group of lamps for tanning apparatus; 1 denotes the structural frame of the said group. The support device 3 for filters according to the present invention is hinged with the side walls 201 of said frame 1. Each lamp 2 comprises a parabolic reflector 102 in the centre of which the bulb 202 provided with a power supply socket 212 is arranged; the parabolic dish is connected to the bottom wall 101 of the frame 1, in which the opening 111 is formed, by means of the flange 112.

The support 3 comprises two sections 103 inside which the filter 203 and the protective screen 303 are arranged; at one end the sections 103 are provided with the plate 403 which carries the pin 423 co-operating with the wall 201 of the frame 1 and which has an end flange 413 which is perpendicular to the plane of the filter 203 and at its end is hinged, by means of the pin 433, with the bar 503 which connects all the end flanges 413 of the devices 3.

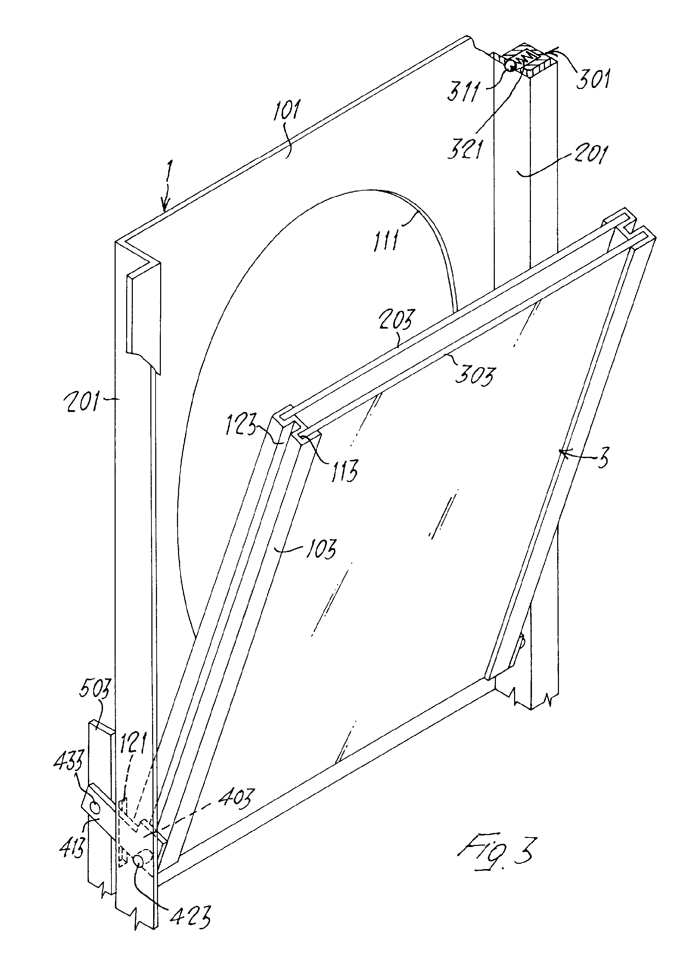

Figure 2 shows the group of lamps according to Figure 1, with the support devices 3 in the open configuration; as can be noted, the bar 503 moves towards the structural frame 1 of the apparatus, limiting in fact the opening movement of the abovementioned devices 3 and at the same time guiding the simultaneous opening of all three devices 3.

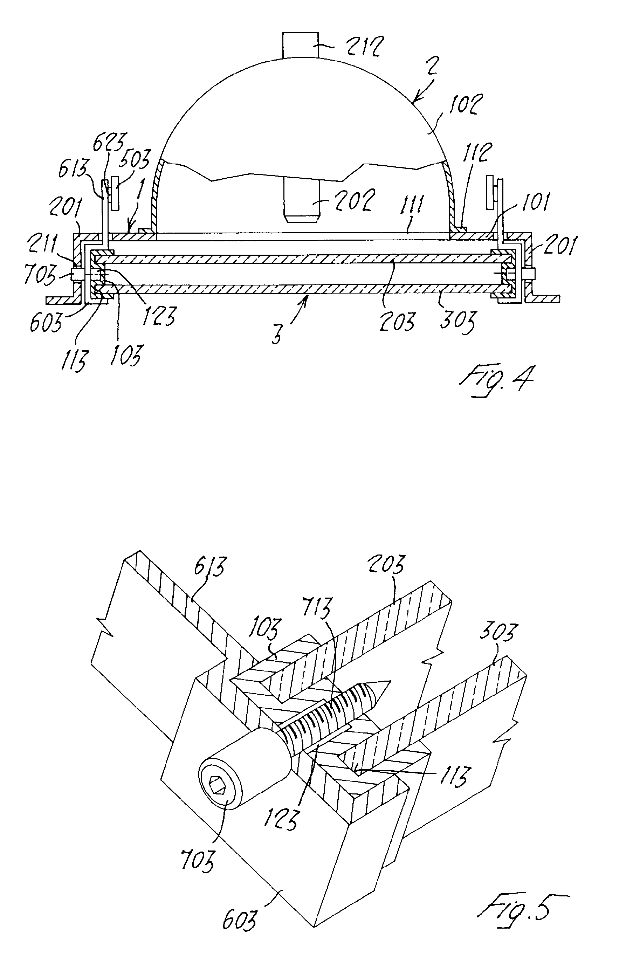

Figure 3 shows a perspective detail illustrating one of the lamp groups shown in Figure 1; identical parts are indicated by identical numbers. The section 103 has the two C-shaped grooves 113 inside which the filter 203 and the protective screen 303 are inserted. The groove 123 is situated in between the two grooves 113 and is intended to co-operate with the locking means 301 which comprise the ball 311 biased by the spring 321. The end flange 413 of the plate 403 moves inside the eyelet 121 formed in the wall 101 of the frame 1.

Figure 4 shows a cross-section of an alternative embodiment of the device according to the present invention; identical parts are indicated by identical numbers. The plate 403 is replaced by the bracket 603 which, as can be seen more clearly in Figure 5, surrounds the section 103. The bracket 603 is provided with the end flange 613 which is joined to the bar 503 by means of the pin 623. The pins which allow hinging of the device 3 on the walls 201 of the frame 1 are in reality the means 703 for fixing the brackets 603 to the sections 103. As can be noted from Figure 5, the threaded end 713 of the said fixing means 703 is inserted inside the groove 123 of the section 103, while the head of the fixing means may be engaged with the hole 211 (see Figure 4) formed in the wall 201 of the structural frame 1.

The operating principle of the device according to the present invention will become clear from the following description. The filter 203 of each lamp group, as well as the protective screen 303, are inserted inside the sections 103 and fixed to them, for example by means of structural silicone or similar fixing means. The assembly thus formed may in this way be conveniently joined to the frame 1, being hinged with it and allowing pivoting thereof through an angle, preferably not less than 30°, so as to allow full access both to the filters and to the bulb 202 of the lamp 2, and not more than 60°, so as not to create an excessive external volume; a preferred angle may be 45° with respect to the plane of the bottom wall 101 of the support frame 1.

Advantageously it is envisaged connecting together the various support frames of the various lamp groups, this being achieved by means of the plates 403, or the brackets 603, and the respective end flanges 413 or 613, which are connected to the bar 503. In this way, opening of the first filter support device 3 will cause opening of all the other devices. The opening angle is determined by the distance of the bar 503 from the frame 1, namely by the length of the end flanges projecting perpendicularly with respect to the sections 103. With this type of solution, accidental opening of the devices may be avoided by providing also only one of the devices (usually the one situated highest along the column) with locking means which may be for example of the type shown in Figure 3.

The constructional variant of Figures 4 and 5 shows different characteristics which make it preferable; firstly the bracket 603 which is positioned around the section 103 prevents any oscillating play thereof, thus making the connection more stable and secure. Secondly the pin which is engaged with the hole 211 in the side wall 201 of the frame 1 is formed by the same fixing means 703 used to connect the bracket 603 to the section 103.

As a result of the device designed in this way, it is possible to perform rapid and simple maintenance of the tanning apparatus lamps, while ensuring at the same time a high degree of operating safety by means of a constructional design which is simple and inexpensive to realise.

## Claims

1. Support device for sun lamp filters used in tanning apparatus, comprising, for each lamp group (2), frame means (103, 403; 603) for supporting at least one filter (203, 303), said supporting frame means (103, 403; 603) being hinged along one side with the structural frame (201) of said apparatus, and said frame (103, 403; 603) for supporting the said filters being movable with respect to said structural frame of said apparatus through a given angle.

2. Device according to Claim 1, in which all the frames (103, 403; 603) for supporting the filter (203, 303) of said lamps (2) are connected together by means of a rigid connection means (503).

3. Device according to Claim 2, in which said frames (103) are provided in the vicinity of the hinged side with an end flange (403; 603) projecting perpendicularly with respect to the plane of said filter (203, 303), each of said end flanges (403; 603) being connected in a pivoting manner to a bar (503).

4. Device according to any one of the preceding Claims 1 to 3, in which means (301) for locking the opening movement of said supporting frame means (103) are envisaged.

5. Device according to any one of the preceding Claims 1 to 4, in which the support frame comprises two sections which are substantially formed as a C inside which two opposite sides of the filter are inserted, the means (403) for connection and hinging with said structural frame (201) of the said apparatus being positioned at one end of each of the said sections (103).

6. Device according to Claims 2, 3 and 5, in which said connection and hinging means comprise a plate (403) provided with a pin (423) intended to co-operate with said structural frame (201) and an end flange (413) projecting perpendicularly with respect to the plane of said filter (203, 303) and able to be joined at the free end to said rigid connection means (503).

7. Device according to any one of Claims 1 to 4, in which the support frame comprises two sections which are substantially formed as a double C inside which two opposite sides of the said filters (203, 303) are inserted, the means (603) for connection and hinging with said structural frame (201) of the said apparatus being positioned at one end of each of the said sections (103).

8. Device according to Claims 2, 3 and 7, in which said connection and hinging means comprise a bracket (603) which is fixed at one end of said sections (103) by means of a fixing means (703) provided with a projecting head able to co-operate with said structural frame (201) of the apparatus, said bracket (603) being provided with an end flange (613) projecting perpendicularly with respect to the plane of said filter (203, 303) and able to be joined at the free end to said rigid connection means (503).

9. Device according to Claim 4 and any one of Claims 7 and 8, in which said locking means comprise a ball (311) arranged projecting from a seat (301) formed in said structural frame (201) of the apparatus, biased with suitable resilient biasing means and co-operating with the groove (123) in one of the said sections (103).
